# EUROPEAN PATENT APPLICATION

(11) **EP 4 082 493 A1**
(43) Date of publication of application: **02.11.2022**
(21) Application number: 21170918.3
(22) Date of filing: 28.04.2021
(51) Int. Cl.: A61F 7/02

(54) **THERMAL CLAY BAG FOR PERSONAL APPLICATION**

(71) Applicant: Siebje, Klaus, 83280-000 Guaraituba (BR); Siebje Mancinelli, Mathias, 89221-300 Joinville (BR); Siebje Mancinelli, Werner, 89280-544 Sao Bento do Sul (BR)
(72) Inventor: SIEBJE, Klaus, 83280-000 Guaraituba (BR)
(74) Representative: Juncosa Miró, Jaime

(57) **Abstract**

Clay-based thermal bag for personal application. The present invention is described as a clay-based thermal bag for personal application which, according to its characteristics, provides a thermal bag in a specific monoblock structure of therapeutic type and based on micronized clays as an active ingredient encased in a flexible cover made of fabric, plastic or any other type of material as protection, in order to provide complete optimization procedures to relieve or even eliminate a wide range of pains in different body parts, combined with a prolonged temperature maintenance time and the absence of effects sides and being based on a thermal bag of high strength, safety and versatility.

## Description

### TECHNICAL FIELD

The present invention relates to thermal bags in general, more specifically to a clay-based thermal bag for personal use.

### DESCRIPTION OF THE INVENTION

The clay-based thermal bag for personal application, according to its general characteristics, has as its basic principle to provide the formation of a thermal bag in specific monoblock structure of therapeutic type and based on micronized clays as an active ingredient encased in a flexible cover made of fabric, plastic or any other type of material as protection, in order to provide, in an extremely practical, safe and comfortable manner, complete optimization procedures to relieve or even eliminate a wide range of aches pain in different body parts, combined to a prolonged temperature maintenance time and the absence of side effects and based on a thermal pack of high strength, safety and versatility.

The clay-based thermal bag for personal application has a specific design and format with an easy access for better adaptation and safety of users, convenience features in handling and functionality, very affordable costs and, due to its general characteristics and dimensions, easily adaptable to a wide range of clays, fabrics, body parts, locations, and users in general, regardless of their characteristics.

The use of hot and/or cold treatments for therapeutic purposes has been widely known in the current state of the art for centuries. Such treatments help treat and relieve several injuries, aches, and pains in the whole body as, for instance, period cramps and baby cramps, toothaches, headaches, backaches, and neck pains. However, there is always a doubt as to which thermal source to use to relieve pains and discomforts or even to help treat lesions.

Hot and cold therapies constitute valuable tools for the treatment of several different types of injuries, although many people do not know much about such alternatives or, most of the times, are in doubt as to which treatment to use, hot or cold. Besides, the mode of application, length and frequency of use also raise many doubts.

To relieve muscle pain in acute injuries/lesions, a cold compress is always welcome, as the purpose of cold compresses is to reduce the temperature at the injured site, curbing the blood flow and constricting deep blood vessels and arteries, thus slowing down the inflammatory process and draining the swelling or bruise.

To relieve the pain in recurring or chronic conditions, like muscle strains or cramps, or even fatigue for overtraining, a hot compress is the most appropriate treatment, as the purpose of hot compresses is to relax the muscles, promoting comfort for stiff or chronic pain muscles and joints.

Besides these doubts, another big obstacle observed by users regards the type of material to be used in hot and cold compresses, among the several different types available, both for the temperature variation causing agent and for the material used in the case.

It should be noted that the most used materials for protection (cases) are various types of rubbers and plastics, and for temperature variation, water, and gels, those being used in liquid (water), solid (ice) and paste form (gel) according to the type of treatment.

It is also worth mentioning that all of these products, regardless of the materials used, still present a high degree of risk in both packaging and transportation, and especially in the procedures used for temperature variations and for the applications they are intended for, because they are highly susceptible to product leakage and even explosions if not well handled.

In this line of action, it has become essential for the users and/or applicators of thermal bags for the most diverse applications, especially those intended for therapeutic purposes on the human body, the structuring of a versatile, practical, safe, and ergonomic vehicle capable of performing thermal applications of heat and/or cold for therapeutic purposes on the various regions of the body with pain and/or trauma.

In an extensive review of the literature, with the purpose of establishing the current state of the art concerning means for thermal applications, primarily for therapeutic purposes on the user's body, object of the present patent of invention, no type of documents in the state of the art were described that relate the specific object claimed in the present patent, i.e., a thermal bag that uses micronized clays coated with a flexible layer for application on the various regions of the body.

Thus, the present invention is characterized by combining components and procedures in a differentiated design, being more efficient and more comfortable and that meets the various requirements the nature of its use demands, i.e., to generate comfort and efficiency in thermal applications for therapeutic purposes on the human body. The concept ensures a thermal bag of great efficiency, functionality, strength, durability, safety, versatility, hygiene, and ergonomics due to the excellent aggregate technical qualities, which provide advantages, improvements, and flexibility in the relief of pains and traumas in the various regions of the user's body for therapeutic purposes and whose general characteristics differ from the other shapes and models widely known in the current state of the art.

More specifically, the present invention consists in the use of a modern, efficient, safe and functional clay-based thermal bag for personal application formed by a set of physicochemical and therapeutic solutions properly incorporated, composing a complete and differentiated thermal bag with exclusive design, great finishing details, beautiful aesthetic aspect and its own characteristics, which incorporates a monoblock structure specific and therapeutic type, of high durability and resistance and containing perfectly integrated and symmetrically disposed micronized or milled clays as active element of hot/cold temperature variation of the thermal bag, and flexible cover as coating and protection element of the thermal bag, in a way to enable the formation of a single, complete and safe set, whose internal and external shapes and dispositions enable the perfect adaptation to a wide range of human body parts in general, being specially designed for these purposes.

In this general conception, the present clay-based thermal bag for personal application, object of the present invention, is entirely based on its simple and robust structure with a minimum required number of components and extremely simple, safe and optimized applicability, combined with fairly practical manufacturing and maintenance procedures so as to generate a practical and efficient thermal bag capable of enabling the user to choose, within a single assembly, the desired temperature to be used in the treatment of different body injuries, that is, to either heat (hot) or cool (cold) the injured part; that is due to the temperature variation feature of the material used - micronized clay, as well as a prolonged temperature maintenance time and ergonomics in the application on the body.

In the same line, the present clay-based thermal bag for personal application is based on the application of components and processes in a differentiated conception, without, however, reaching a high degree of sophistication and complexity, making it possible to solve some of the main drawbacks of the other forms and models widely known by the current state of the art and employed in the treatment for therapeutic purposes on the body, which are situated in a working range in which are very often difficulty of use and application, low efficiency and performance, great deterioration and fragility, little durability and resistance, low versatility, high cost, simple adaptations, no comfort, high volume and general weight, little flexibility.

The present invention of a clay-based thermal bag for personal application comprises a complete thermal bag with its own characteristics, which incorporates a therapeutic-type specific monoblock structure of high durability and resistance, internal and external shapes and arrangements that adapt to a wide range of human body parts in general, and containing perfectly integrated and symmetrically arranged among themselves a portion of micronized or ground clays as the active principle of hot/cold temperature variation of the thermal bag, and a flexible cover as lining and protection of the thermal bag.

The clay-based thermal bag for personal application has fully integrated components, it can be assembled and disassembled quickly, nothing comes loose or breaks off or gets warped, achieving a high index of performance and efficiency, combined with high durability and complete safety. Once fully integrated, the components are completely locked and united, thus preventing them from loosening when in use, making the assembly fully available for thermal application procedures for therapeutic purposes onto different parts of the human body. The thermal bag can be thus used without concerns of any nature, mainly regarding the safety and durability of its components, as well as the safety of its users.

In view of the foregoing, the thermal bag will be well-received by manufacturers, sellers and users of thermal bags for personal application in general, since the clay-based thermal bag for personal application has many advantages, such as: great safety, reliability and flexibility in its application; great efficiency and performance due to its general conception; great comfort, convenience and safety for the users; great overall strength and durability, combined with low or no wear of the assembly as a whole; fully accessible costs which provides an optimum cost-benefit ratio; practical and safe use by any users; fully compatible weight and overall dimensions; high operating range; proper and direct adaptation to different types of pillows; great mobility and flexibility of the assembly; high operating ergonomics; high range of benefits; complete hygienization; high capacity of holding desired temperature; concept of multifunctionality in thermal applications for therapeutic purposes - hot or cold; based on the dry treatment concept - no need for aftercare and allows for multiple applications; absence of any side effects; no leakage of the active ingredient; and the certainty of having a product that fully meets the current rules and regulations required to its application.

All such attributes classify the clay-based thermal bag for personal application as a fully versatile, efficient, practical and safe way to be used in the dry application procedures of a wide range of thermal applications for therapeutic purposes onto different body parts, by the most diverse types of users and in various types of locations, regardless of the general characteristics they may have, being also easy to use and handle, combined with great performance and excellent general characteristics; nevertheless, the sizes, dimensions and quantities may vary according to the needs of each application.

## Claims

1. Clay-based thermal bag for personal application, **characterized in that** it comprises a thermal bag that incorporates a therapeutic type monoblock structure containing integrated and symmetrically arranged between them a portion of micronized or ground clay as an active ingredient and a flexible coating for protecting the thermal bag.
